(19) Europäisches Patentamt
European Patent Office
Office européen des brevets

(11) Numéro de publication : 0 348 254 B1

(12) # FASCICULE DE BREVET EUROPEEN

(45) Date de publication du fascicule du brevet : **23.06.93 Bulletin 93/25**

(51) Int. Cl.$^5$ : **C07C 69/743**

(21) Numéro de dépôt : **89401424.0**

(22) Date de dépôt : **25.05.89**

(54) **Procédé de préparation de composés norpyréthriques.**

(30) Priorité : **25.05.88 FR 8806934**

(43) Date de publication de la demande : **27.12.89 Bulletin 89/52**

(45) Mention de la délivrance du brevet : **23.06.93 Bulletin 93/25**

(84) Etats contractants désignés : **CH DE FR GB IT LI NL**

(56) Documents cités :
**EP-A- 0 107 570**
**TETRAHEDRON, tome 42, no. 9, 1986, pages 2475-2484, Pergamon Press, Oxford, GB; M. JULIA et al, "Organic synthesis with sulfones XXXVIII on the mechanism of the stereo-specific hydrogenolysis of vinylic sulfones by sodium dithionite"**

(56) Documents cités :
**TETRAHEDRON LETTERS, tome 23, no. 32, 1982, pages 3265-3266, Pergamon Press, Oxford, GB; J. BREMNER et al, "Synthèses à l'aide de sulfones (XXIV). Synthèse stéréosélective d'oléfines par hydrogénolyse des vinylsulfones"**

(73) Titulaire : **ROUSSEL-UCLAF**
**35, Boulevard des Invalides**
**F-75007 Paris (FR)**

(72) Inventeur : **Babin, Didier**
**22, rue de la Grenouillette**
**F-78180 Montigny (FR)**
Inventeur : **Demoute, Jean-Pierre**
**65, Avenue Foch**
**F-93360 Neuilly Plaisance (FR)**
Inventeur : **Tessier, Jean**
**30, rue Jean Moulin**
**F-94300 Vincennes (FR)**

(74) Mandataire : **Tonnellier, Marie-José et al**
**111, route de Noisy B.P. no 9**
**F-93230 Romainville (FR)**

EP 0 348 254 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de dérivés norpyréthriques.

La synthèse d'oléfines avec inversion de configuration par hydrogénolyse des sulfones vinyliques correspondants a été décrite dans Tetrahedron Letters, Vol. 23, N° 32, pp. 3265 - 3266, 1982 et Tetrahedron, Vol. 42, N° 9, pp. 2475 à 2482, 1986.

L'invention a pour objet un procédé de préparation des produits formule (V) :

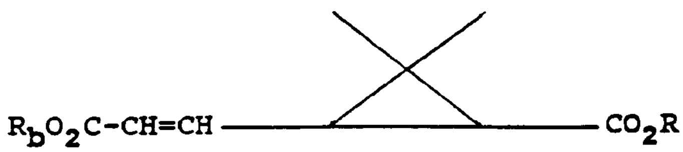

(V)

dans laquelle $R_b$ représente un radical alkyle linéaire, ramifié ou cyclisé saturé ou insaturé, renfermant jusqu'à 18 atomes de carbone, éventuellement substitué par un ou plusieurs groupes fonctionnels, ou $R_b$ représente un radical aryle renfermant jusqu'à 14 atomes de carbone, éventuellement substitué par un ou plusieurs groupes fonctionnels et R représente un atome d'hydrogène, un radical alkyle renfermant jusqu'à 8 atomes de carbone ou

- soit un groupe

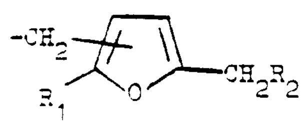

dans lequel le substituant $R_1$ représente un atome d'hydrogène ou un radical méthyle et le substituant $R_2$ un aryle monocyclique ou un groupe 2-propynyle et notamment un groupe (5-benzyl 3-furyl) méthyle,

- soit un groupe

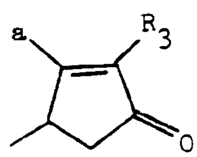

dans lequel a représente un atome d'hydrogène ou un radical méthyle et $R_3$ représente un radical organique aliphatique comportant de 2 à 6 atomes de carbones et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$,

- soit un groupe

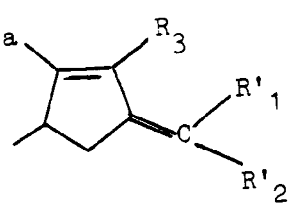

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alkyle renfermant de 1 à 6 atomes de carbone, un radical aryle comportant de 6 à 10 atomes de carbone, un groupe alkyloxycarbonyle comportant de 2 à 5 atomes de carbone,

- soit un groupe

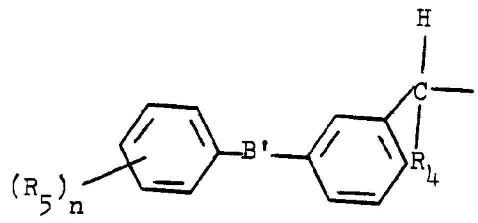

dans lequel B' représente un atome d'oxygène ou de soufre, un groupe carbonyle, méthylène, sulfinyle ou sulfonyle et $R_4$ représente un atome d'hydrogène, un radical cyano, un radical méthyle, un radical carbamoyle, un radical thiocarbamoyle ou un radical éthynyle, $R_5$ représente un atome d'halogène ou un radical méthyle et n représente un nombre égal à 0, 1 ou 2, et notamment le groupe 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thiocarbamoyle 3-phénoxy benzyle,

- soit un groupe

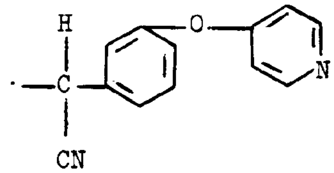

- soit un groupe

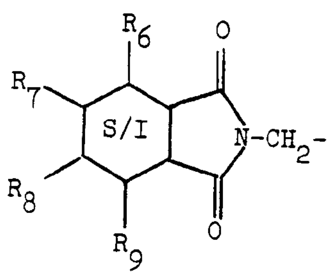

dans lequel les substituants $R_4$, $R_7$, $R_4$, $R_9$ représentent un atome d'hydrogène, un atome de chlore, ou un radical méthyle et dans lequel S/I symbolise un cycle aromatique ou un cycle analogue dihydro ou tétrahydro,

- soit un groupe

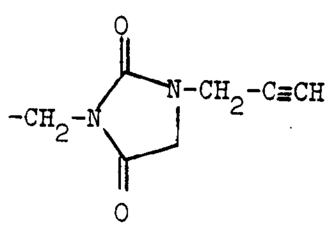

- soit un groupe

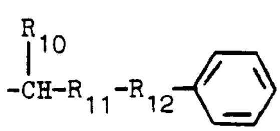

dans lequel $R_{10}$ représente un atome d'hydrogène ou un radical cyano, $R_{12}$ représente un radical méthylène ou un atome d'oxygène, $R_{11}$ représente un radical thiazolediyle ou thiadiazolediyle lié à

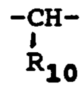

par l'une quelconque des positions disponibles du cycle et à $R_{12}$ par l'atome de carbone compris entre l'atome de soufre et un atome d'azote,

- soit un groupe

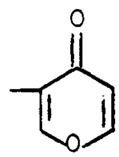

- soit un groupe

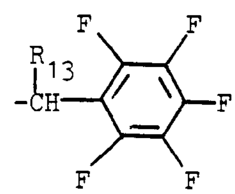

dans lequel $R_{13}$ représente un atome d'hydrogène ou un radical cyano,

- soit un groupe

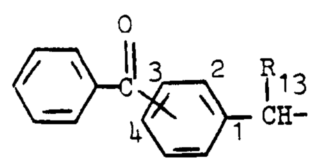

dans lequel $R_{13}$ est défini comme ci-dessus, et le radical benzoyle est en position 3 ou 4,

- soit un groupe

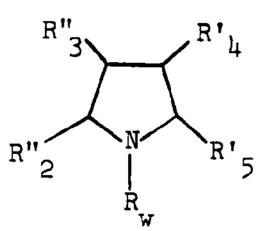

dans lequel l'un des radicaux $R''_2$ ou $R''_3$ représente un radical -CHZ, Z représentant un atome d'hydrogène, un groupe cyano, éthynyle, trifluorométhyle ou un radical alkyle renfermant de 1 à 3 atomes de carbone et celui des radicaux $R''_2$ ou $R''_3$ qui ne représente pas un radical -CHZ ainsi que les radicaux $R'_4$ et $R'_5$ identiques ou différents les uns des autres, représentent un atome d'hydrogène, un atome d'halogène, un radical alkyle renfermant jusqu'à 18 atomes de carbone, un radical aryle renfermant jusqu'à 14 atomes de carbone, un radical aralkyle renfermant jusqu'à 18 atomes de carbone, un radical cyano, un radical trifluorométhyle, un radical alcoxycarbonyle renfermant jusqu'à 8 atomes de carbone, un groupe nitro, un radical alkyloxy renfermant jusqu'à 8 atomes de carbone, un radical :

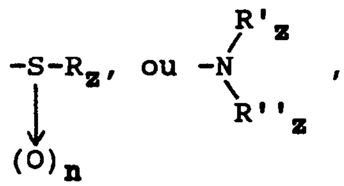

n étant égal à 0,1 ou 2 et les radicaux $R_z$, $R'_z$ et $R''_z$ représentant un radical alkyle renfermant de 1 à 8 atomes de carbone, les radicaux $R'_4$ et $R'_5$ pouvant aussi former un homocycle carboné saturé ou insaturé renfermant jusqu'à 8 atomes de carbone et $R_w$ représente :

- soit un radical

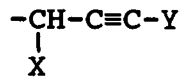

dans lequel X et Y, identiques ou différents l'un de l'autre, représentent un atome d'hydrogène, un atome d'halogène, un radical alkyle renfermant de 1 à 8 atomes de carbone ou un radical aryle renfermant jusqu'à 14 atomes de carbone

- soit un radical

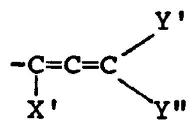

ou
un radical

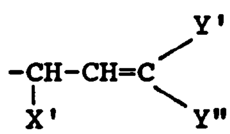

dans lequel X', Y' et Y", identiques ou différents les uns des autres, représentent l'une des valeurs indiquées ci-dessus pour X et Y ;

- soit un radical

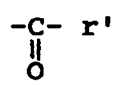

dans lequel r' représente un atome d'hydrogène, un radical alkyle renfermant jusqu'à 14 atomes de carbone, un radical aralkyle renfermant jusqu'à 18 atomes de carbone, un radical trifluorométhyle, un radical alcoxycarbonyle, un radical alkyloxy renfermant jusqu'à 8 atomes de carbone, un radical alkylthio

- soit un radical

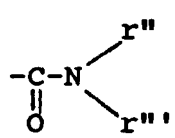 ,

dans lequel r" et r‴, identiques ou différents l'un de l'autre, représentent un atome d'hydrogène, un radical alkyle renfermant de 1 à 18 atomes de carbone, un radical aryle renfermant jusqu'à 14 atomes de carbone, un radical aralkyle renfermant jusqu'à 18 atomes de carbone, un radical trifluorométhyle, un

radical alcoxycarbonyle renfermant jusqu'à 8 atomes de carbone ou un radical alkyloxy renfermant jusqu'à 8 atomes de carbone.

- soit un groupe

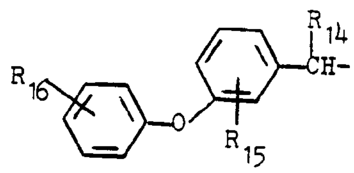

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

- soit un groupe

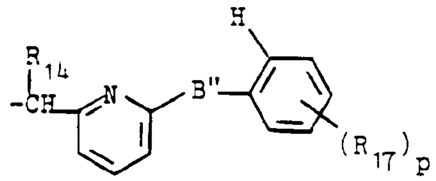

dans lequel $R_{14}$ est défini comme précédemment, B" représente un atome d'oxygène ou de soufre, p est un nombre égal à 0, 1 ou 2, $R_{17}$ représente un groupe alkyle renfermant de 1 à 4 atomes de carbone, alkylthio renfermant de 1 à 4 atomes de carbone, alkylsulfonyle renfermant de 1 à 4 atomes de carbone, trifluorométhyle, chloro, fluoro ou bromo et quand p est égal à 2, soit $(R_{17})_p$ représente un groupe méthylènedioxy lié au noyau phényle aux positions 3 et 4, soit chacun des $R_{17}$ représente indépendamment un des groupes cités ci-dessus, caractérisée en ce que l'on soumet un composé de formule (I) :

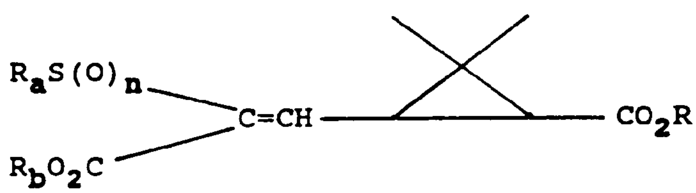

(I)

dans laquelle n représente le nombre 0, 1 ou 2, $R_a$ représente un radical alkyle linéaire, ramifié ou cyclisé, saturé ou insaturé, renfermant jusqu'à 18 atomes de carbone ou un radical aryle renfermant jusqu'à 14 atomes de carbone, éventuellement substitué par un ou plusieurs groupes fonctionnels, $R_b$ et R sont tels que définis ci-dessus, à l'action de l'anion $S_2O_4^{--}$ ou toute autre source de $HSO_2^-$ ou $SO_2^{--}$, pour obtenir le composé de formule (IV) correspondant :

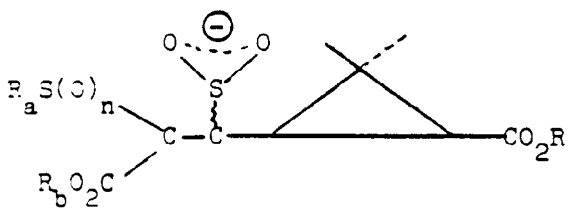

(IV)

dans laquelle $R_a$, $R_b$ et R conservent la même signification que précédemment, que l'on isole si désiré ou qui se décompose spontanément pour donner le composé de formule (V) correspondant.

Lorsque $R_a$, $R_b$ ou R représente un radical alkyle saturé, linéaire ou ramifié, il s'agit de préférence d'un radical méthyle, éthyle, n-propyle, isopropyle, n-butyle, sec-butyle, isobutyle, n-pentyle, n-hexyle, tert-butyle, tert-pentyle ou néopentyle.

Lorsque $R_a$, $R_b$ ou R représente un radical cyclique, il s'agit de préférence d'un radical cyclopropyle, cyclobutyle, cyclopentyle ou cyclohexyle, d'un radical alkyle linéaire ou ramifié portant un radical cyclique ou

d'un radical cyclopropyle, cyclobutyle, cyclopentyle ou cyclohexyle substitué par un ou plusieurs radicaux alkyles, par exemple les radicaux 1-méthylcyclobutyle, 1-méthylcyclopentyle, 1-méthylcyclohexyle ou 2,2,3,3 tétraméthyl cyclopropyle.

Lorsque $R_a$, $R_b$ ou R représente un radical alkyle insaturé, il s'agit d'un radical éthylénique, comme par exemple les radicaux vinyle ou 1,1-diméthyl alkyle ou d'un radical acétylénique, comme par exemple les radicaux éthynyle ou propynyle.

Lorsque $R_b$ représente un radical alkyle substitué par un ou plusieurs groupes fonctionnels, on entend de préférence par alkyle un radical renfermant de 1 à 8 atomes de carbone comme, par exemple, les radicaux méthyle, éthyle, propyle, isopropyle, n-butyle, isobutyle ou tert-butyle.

Lorsque $R_b$ représente un radical alkyle substitué par un ou plusieurs groupes fonctionnels, on entend de préférence par groupe fonctionnel, un atome d'halogène, un groupe hydroxy ou mercapto, un groupe alkyloxy ou alkylthio renfermant de 1 à 8 atomes de carbone, ou un groupe nitro,

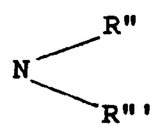

dans lequel R" et R''', identiques ou différents, représentent un atome d'hydrogène ou un radical alkyle renfermant de 1 à 8 atomes de carbone, un groupe cyano, $SO_3H$ ou $PO_3H_2$ ou un groupe $alk_1SO$, $alk_2SO_2$ ou $alk_3SO_3$ dans lesquels $alk_1$, $alk_2$ et $alk_3$ représentent des radicaux alkyles renfermant de 1 à 18 atomes de carbone.

$R_b$ peut représenter également un radical alkyle substitué par un radical aryle comme, par exemple, le radical benzyle ou le radical phénéthyle, lui-même éventuellement substitué par un ou plusieurs groupes hydroxy, alkyloxy ou alkyle, renfermant de 1 à 8 atomes de carbone, par un ou plusieurs halogènes ou groupes trifluorométhyle, trifluorométhoxy, trifluorométhylthio, ou par un groupe méthylènedioxy.

$R_b$ peut représenter également un radical alkyle substitué sur deux carbones adjacents par un groupe méthylènedioxy ou substitué par un groupe 2-tétrahydropyrannyloxy.

Lorsque $R_b$ représente un radical alkyle substitué par un ou plusieurs groupes fonctionnels on peut citer comme valeurs préférées de $R_b$ :

$-(CH_2)_n-C(Hal)_3$ dans lequel n est un entier de 1 à 8 et Hal est un atome d'halogène, par exemple les radicaux 2,2,2-trichloroéthyle, 2,2,2-trifluoroéthyle, 3,3,3-trichloropropyle ou 3,3,3-trifluoropropyle.

$-(CH_2)_{n1}-CH(Hal)_2$ dans lequel Hal est défini comme ci-dessus et $n_1$ est un nombre de 0 à 8, par exemple les radicaux 2,2-dichloroéthyle, 2,2-difluoroéthyle ou difluorométhyle.

$-(CH_2)_n-CH_2(Hal)$ dans lequel n et Hal sont définis comme ci-dessus, par exemple radicaux 2-chloroéthyle ou 2-fluoroéthyle.

$-C[C(Hal)_3]_3$ dans lequel Hal est défini comme ci-dessus, par exemple les radicaux 2,2,2-trifluoro 1,1-bis(trifluorométhyl) éthyle ou 2,2,2-trichloro 1,1-bis(trifluorométhyl) éthyle.

- les radicaux 2,2,2-trifluoro 1-trifluorométhyl 1-méthyl éthyle, 2,2,2-trifluoro 1,1-diméthyle éthyle ou 1-trifluorométhyl 1-méthyl propyle.
- les radicaux 2,2,2-trifluoro 1-méthyl éthyle ou 2,2,2-trifluoro, 1-trifluorométhyl éthyle.
- les radicaux 1-cyano 1-méthyl éthyle, 1-cyano éthyle ou

$-(CH_2)_n-CN$ dans lequel n est défini comme précédemment.

- les radicaux 2,2,2-trihalo 1-cyano éthyle comme par exemple le radical 2,2,2-trichloro 1-cyano éthyle.

$-(CH_2)_n-OR'$, dans lequel n est défini comme précédemment et R' représente un atome d'hydrogène ou un radical alkyle linéaire ou ramifié, comportant de 1 à 8 atomes de carbone, par exemple les radicaux méthoxyméthyle, 2-méthoxyéthyle, 2-éthoxyéthyle ou 2-hydroxyéthyle.

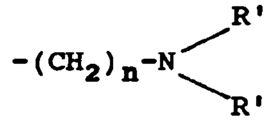

dans lequel n et R' sont définis comme précédemment et les deux radicaux R' peuvent être différents entre eux, par exemple les radicaux 2-(méthylamino) éthyle, 2-(diméthylamino) éthyle ou 2-(éthyl méthylamino) éthyle

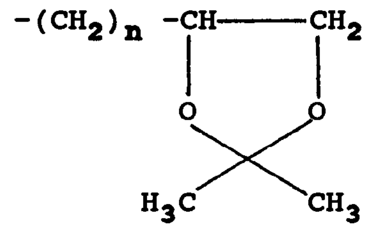 ,

dans lequel n est défini comme précédemment, par exemple le radical

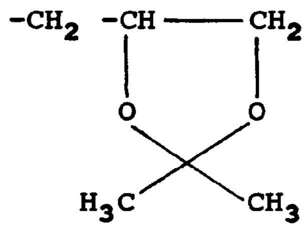 ,

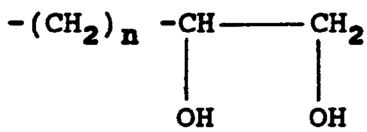

dans lequel n est défini comme précédemment, par exemple le radical 2,3-dihydroxypropyle

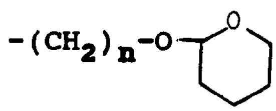

dans lequel n est défini comme précédemment, par exemple les radicaux (2-tétrahydropyrannyloxy) méthyle ou 2-(2-tétrahydropyrannyloxy) éthyle

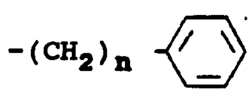

dans lequel n est défini comme précédemment, par exemple les radicaux benzyle ou phénéthyle,

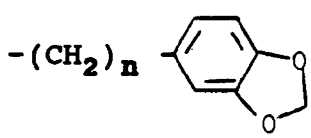

dans lequel n est défini comme précédemment, par exemple le radical pipéronyle.

Lorsque $R_b$ représente un radical aryle éventuellement substitué, il s'agit de préférence du radical phényle ou du radical phényle substitué par un ou plusieurs groupes hydroxy alkyloxy renfermant de 1 à 8 atomes de carbone, ou par un halogène ou un groupe trifluorométhyle, trifluorométhoxy ou trifluorométhylthio.

Dans un mode de réalisation préféré de l'invention, on part des composés dans lesquels n représente le nombre 2, ceux dans lesquels $R_a$ représente un radical tert-butyle ou encore ceux dans lesquels $R_a$ représente un radical phényle, éventuellement substitué par un radical $Oalc_1$, $N(alc_2)_2$, $alc_3$, $alc_1$, $alc_2$ et $alc_3$ représentant un radical alkyle renfermant jusqu'à 8 atomes de carbone.

Comme valeurs préférées de $R_b$, on peut citer les radicaux alkyle linéaire ou ramifié, saturé, renfermant jusqu'à 6 atomes de carbone, éventuellement substitué par un ou plusieurs atomes d'halogène comme par

exemple un radical tertbutyle ou un radical

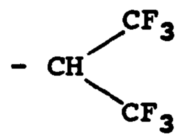

Comme valeurs préférées de R, on peut citer le radical :

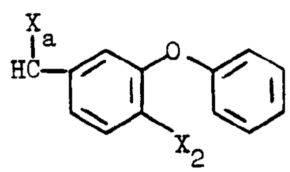

dans lesquels $X_a$ représente un atome d'hydrogène, un radical méthyle, cyano ou éthynyle, et $X_2$ représente un atome d'hydrogène ou un atome de fluor ou le radical

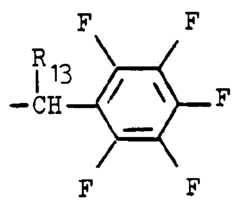

dans lequel $R_{13}$ conserve la même signification que ci-dessus.

Dans un mode de réalisation de l'invention, on part des composés de formule (I) dans lesquels la copule cyclopropanique est de structure (1R cis) ou (1R trans), ainsi que les mélanges des composés (1R cis) et (1R trans). Les composés préférés de l'invention sont ceux dans lesquels la configuration au niveau du cyclopropane est (1R cis).

Les produits de formule (V) ainsi préparés sont des produits connus d'une façon générale, décrits par exemple dans les brevets européens 0038271, 0041021, 0048186. Comme il est indiqué dans ces brevets, les produits de formule (V) les plus intéressants d'un point de vue biologique sont les produits dans lesquels la géométrie de la double liaison est Z. La partie expérimentale exposée ci-après montre clairement que les produits de formule (V) préparés selon l'application de l'invention sont majoritairement des produits de géométrie Z.

Les produits de formule (I) sont connus de façon générale et sont préparés comme décrits dans la demande de brevet européen n° 0107570 et dans les préparations suivantes.

Dans un mode de réalisation préféré de l'invention, l'agent réducteur est le dithionite de sodium, $Na_2S_2O_4$, dans un mélange d'eau et de solvant organique avec éventuellement un agent de transfert de phase.

Comme agent de transfert de phase, on peut citer les sels de tricaprylméthylammonium, de tétrabutylammonium, de triéthylbenzylammonium, ou tout autre sel d'ammonium quaternaire ainsi que les sels de phosphonium.

Lorsque l'on opère en l'absence d'un agent de transfert de phase, la réaction est plus lente et l'on peut si désiré, après acidification du milieu réactionnel par exemple à l'aide d'un acide minéral tel que l'acide chlorhydrique, faire agir un agent de méthylation tel que le diazométhane pour isoler le produit de formule (IV) sous forme de sulfimate de méthyle.

Les produits de formule (IV) sont des produits nouveaux et l'invention a également pour objet les produits de formule (IV) à titre d'intermédiaire nouveau.

Les exemples suivants illustrent l'invention sans toutefois la limiter.

**PREPARATION 1 : (1R cis) -3-[(E)-3-tert-butoxy 2-(tert-butylsulfonyl) 3-oxo 1-propényl] 2,2-diméthyl cyclopropanecarboxylate de (S)-alpha-cyano 3-phénoxy benzyle.**

Stade A : (1R cis)-4-[2-tert-butoxy 1-(tert-butylsulfonyl) 2-oxo éthyl] 6,6-diméthyl 2-oxo 3-oxabicyclo-[3.1.0]hexan-2-one.

On ajoute à 20°C, 3,5 g d'hydrure de sodium à 50 % dans l'huile dans une solution renfermant 17,25 g de (tert-butyl sulfonyl) acétate de tert-butyle et 175 ml de tétrahydrofuranne. On agite à 20°C pendant une demi-heure. On ajoute 10 ml de diméthylformamide et maintient sous agitation pendant deux heures. On ajoute ensuite 10,37 g de la lactone de l'acide (1R,cis)-(dihydroxyméthyl) 2,2-diméthyl cyclopropanecarboxylique, agite pendant 18 heures à 20°C puis chauffe pendant 5 heures à 50°C. On verse sur une solution saturée de phosphate acide de sodium. On décante, isole la phase organique et concentre. On purifie le produit obtenu par chromatographie sur silice, en éluant à l'aide du mélange hexane acétate d'éthyle (1-1). On obtient ainsi 9,2 g de produit recherché.

Spectre RMN ($CDCl_3$, 60 MHz)
1,22 ppm : groupes méthyl géminés du cycle
1,5 ppm : groupes tert-butyl
1,95-2,05 ppm : hydrogène en position 1 du cycle
2,43 ppm : hydrogène en position 3 du cycle
4,18 ppm à 4,47 ppm : O-CH-CH-

Stade B : (1R cis) -3-[(E)-3-tert-butoxy 2-(tert-butylsulfonyl) 3-oxo 1-propényl] 2,2-diméthyl cyclopropanecarboxylate de (S)-alpha-cyano 3-phénoxy benzyle.

On ajoute 1,87 g d'alcool (S)-alpha-cyano 3-phénoxy benzylique dans une solution renfermant 3 g de produit préparé au stade A, dans 25 ml de chlorure de méthylène. On refroidit à + 10°C et ajoute goutte à goutte en 30 minutes 1,75 g de dicyclohexylcarbodiimide et 50 mg de diméthylamino pyridine dans 50 ml de chlorure de méthylène. On laisse sous agitation pendant 18 heures à 20°C. On filtre et concentre. On obtient 5 g d'un produit que l'on chromatographie sur silice en éluant par le mélange hexane-éther isopropylique(4-6). On isole 3,5 g de produit recherché fondant à 102°C.

**PREPARATION 2 : (1R cis) -3-[(E)-3-tert-butoxy 2-(tert-butylsulfonyl) 3-oxo 1-propényl] 2,2-diméthyl cyclopropanecarboxylate de 3-phénoxy benzyle.**

On ajoute 3,5 g d'alcool m-phénoxybenzylique dans une solution renfermant 6,3 g de (1R cis)-4-[2-tert-butoxy 1-(tert-butylsulfonyl) 2-oxo éthyl] 6,6-diméthyl 2-oxo 3-oxabicyclo[3.1.0]hexan-2-one (cf stade A de l'exemple 1) et 50 ml de chlorure de méthylène. On refroidit à + 10°C et ajoute en une demi-heure 3,6 g de dicyclohexylcarbodimide et 120 mg de diméthylaminopyridine en solution dans 60 ml de chlorure de méthylène. On maintient sous agitation le mélange réactionnel pendant 18 heures à 20°C. On filtre et concentre. On obtient 10 g d'huile qui donne après chromatographie en éluant avec le mélange hexane éther isopropylique (4-6), 6,35 g de produit recherché fondant à 72°C.

**PREPARATIONS 3 à 14 : en opérant selon l'un des exemples précédents, on prépare les produits suivants :**

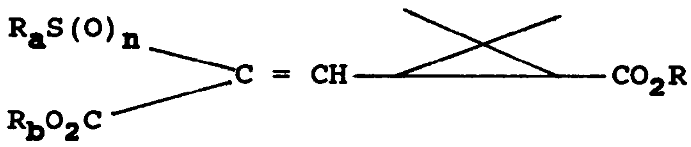

**En série 1 Rcis**

| $R_aS(O)_n$ | $R_b$ | R |
|---|---|---|
| $C_6H_5SO_2$ | tbu | $CH_3$ |
| 4-MeO $C_6H_4SO_2$ | tbu | $CH_3$ |
| 4-$NH_2$ $C_6H_4SO_2$ | tbu | $CH_3$ |
| 4-$NMe_2C_6H_4SO_2$ | tbu | $CH_3$ |
| 4-MeO $C_6H_4SO_2$ | tbu | 3-phénoxy benzyl |
| 2-MeO $C_6H_4SO_2$ | tbu | 3-phénoxy benzyl |
| 2-MeO $C_6H_4SO$ | tbu | 3-phénoxy benzyl |
| $C_6H_5SO$ | tbu | 3-phénoxy benzyl |
| $C_6H_5SO_2$ | Et | $CH_3$ |
| $C_6H_5SO_2$ | Me | $CH_2$ 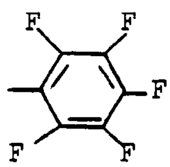 |
| $CH_3SO_2$ | tbu | $CH_3$ |

**et en série 1R trans**

| $R_aS(O)_n$ | $R_b$ | R |
|---|---|---|
| $C_6H_5SO$ | tbu | 3-phenoxybenzyl |

EXEMPLE 1 : (1R cis)-3-[(Z)-3-tert-butoxy 3-oxo 1-propényl] 2,2-diméthyl cyclopropanecarboxylate de (S)-alpha-cyano 3-phénoxy benzyle.

On ajoute 9 ml de tampon pH 8,5 obtenu en mélangeant 8,3 g de phosphate acide de potassium, 50 ml d'eau et 56 ml d'une solution normale de soude, dans une solution renfermant 900 mg de (1R cis) -3-[(E)-3-tert-butoxy 2-(tert-butylsulfonyl) 3-oxo 1-propényl] 2,2-diméthyl cyclopropanecarboxylate de (S)-alpha-cyano 3-phénoxy benzyle, 9 ml d'une solution de cyclohexane renfermant 162 g/l de chlorure de tricaprylméthylammonium ou aliquat 336. On introduit ensuite 560 mg de dithionite de sodium. On maintient le mélange réactionnel sous agitation pendant 15 minutes, à 20°C. On chauffe 1 h 30 mn au reflux. On refroidit. On extrait au chlorure de méthylène, on lave à l'eau. On sèche et évapore sous vide. On obtient 2 g de produit, que l'on chromatographie sur silice en éluant par le mélange cyclohexane-acétate d'éthyle 8-2. On isole ainsi :
400 mg d'isomère Z
20 mg d'isomère E.

Le rapport $\frac{Z}{E}$ est donc de 95/5.

Spectre RMN 60MHz

| | |
|---|---|
| 1,23 - 1,25 ppm | : groupes Méthyl géminés du cycle |
| 1,47 ppm | : tert-butyl |
| 1,95 - 2,09 ppm | : Hydrogène en positions 1 du cycle |
| 3,1 - 3,26 - 3,41 ppm | : Hydrogène en position 3 du cycle |
| 5,98 ppm | : hydrogène benzylique |

| | |
|---|---|
| 5,68 - 5,87 ppm | : Hydrogène en position 2 du groupe 1-propényl (Z) |
| 6,2 - 6,55 ppm | : Hydrogène en position 1 du groupe 1-propényl |
| 6,88 à 7,5 ppm | : les hydrogènes aromatiques. |

EXEMPLE 2 : (1R cis)-3-[(Z)-3-tert-butoxy 3-oxo 1-propényl] 2,2-diméthyl cyclopropanecarboxylate de 3-phénoxy benzyle.

On ajoute 3 ml d'eau distillée, 210 mg de dithionite de sodium et 126 mg de bicarbonate de sodium dans une solution renfermant 320 mg de (1R cis) -3-[(E)-3-tert-butoxy 2-(tert-butylsulfonyl) 3-oxo 1-propényl] 2,2-diméthyl cyclopropanecarboxylate de 3-phénoxy benzyle et 3 ml d'une solution de cyclohexane renfermant 162 g/l de chlorure de tricaprylméthylammonium ou aliquat 336. On chauffe le mélange obtenu au reflux pendant 1 h 15.
On refroidit, extrait la phase organique au chlorure de méthylène, lave à l'eau. On sèche et évapore sous pression réduite. On obtient 0,7 g de produit brut que l'on chromatographie sur silice en éluant avec le mélange hexane acétate d'éthyle 9-1. On isole 157 mg d'ester Z et 13 mg d'ester E.
Le rapport $\frac{Z}{E}$ du produit obtenu est donc environ 92/8.

EXEMPLE 3A : (1R cis)-3-[(Z)-3-tert-butoxy 3-oxo 1-propényl] 2,2-diméthyl cyclopropanecarboxylate de 3-phénoxy benzyle.

On opère comme dans l'exemple 2, mais le milieu réactionnel est homogène et est constitué par un mélange THF, méthanol, eau et sans agent de transfert.
On chauffe le mélange réactionnel 3 heures à 60°C. On obtient le produit recherché ; rendement = 69 % ;
rapport $\frac{Z}{E}$ = 95/5

Cet exemple illustre l'invention dans le cas où l'on opère en phase homogène.

EXEMPLE 3B : 1R (1alpha, 3alpha) 2,2-diméthyl 3-[1-méthoxysulfinyl 2-tert-butylsulfonyl 3-(1,1-diméthyléthoxy) 3-oxo propyl] cyclopropane carboxylate de (3-phénoxy phényl) méthyle.

On ajoute 27 ml d'eau et 27 ml de méthanol dans une solution comprenant 3 g de (1R cis) 3-[(E) 3-tert-butoxy 2-(tertbutylsulfonyl) 3-oxo 1-propényl] 2,2-diméthyl cyclopropane carboxylate de (S) (3-phénoxy phényl) méthyle dans 55 ml de tétrahydrofuranne. On ajoute goutte à goutte en 30 minutes, à 20 °C, 1,34 g de dithionite de sodium dissous dans 30 ml d'eau. On agite 1 heure la solution comprenant l'ion sulfinate puis élimine sous pression réduite, le méthanol et le tétrahydrofuranne. On refroidit à +5°C, la phase aqueuse restante, acidifie à pH=1 à l'aide de 0,8 ml d'acide chlorhydrique 11,8 N, extrait à l'éther éthylique, lave à l'aide d'une solution aqueuse de chlorure de sodium, sèche et concentre à 25°C sous pression réduite. On dissout 3,4 g de résidu dans 40 ml de chlorure de méthylène, refroidit à +10°C, ajoute goutte à goutte 40 ml d'une solution de diazométhane dans le chlorure de méthylène, agite 15 minutes et concentre à sec sous pression réduite. On chromatographie le résidu sur silice (éluant hexane-acétate d'éthyle (7-3)) et obtient 2 g de produit attendu (mélange de 4 isomères). Cet exemple illustre l'isolation de l'intermédiaire de formule (IV) dans le cas où l'on opère en phase homogène.

EXEMPLES 4 à 12 :

On a préparé les produits de formule (V), en opérant comme dans les exemples d'application 1 et 2 en suivant le schéma réactionnel.

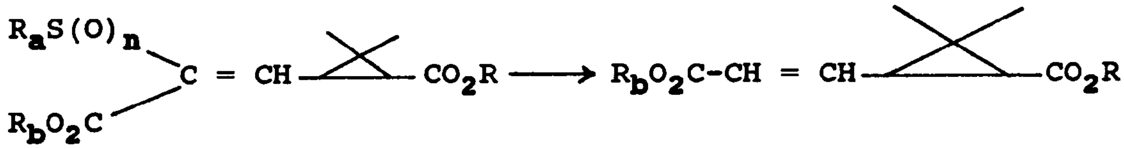

I V

En série 1R cis

| $R_aS(O)_n$ | $R_b$ | R | Z/E | Rendement |
|---|---|---|---|---|
| $C_6H_5SO_2$ | tbu | $CH_3$ | 60/40 | 71 % |
| 4-MeO $C_6H_4SO_2$ | tbu | $CH_3$ | 74/26 | 70 % |
| 4-$NH_2$ $C_6H_4SO_2$ | tbu | $CH_3$ | 71/29 | 66 % |
| 4-$NMe_2C_6H_4SO_2$ | tbu | $CH_3$ | 65/35 | - |
| 4-MeO $C_6H_4SO_2$ | tbu | 3-phénoxy benzyl | 78/22 | 70 % |
| 2-MeO $C_6H_4SO_2$ | tbu | 3-phénoxy benzyl | 85/15 | 60 % |
| 2-MeO $C_6H_4SO$ | tbu | 3-phénoxy benzyl | 80/20 | - |
| $C_6H_5SO$ | tbu | 3-phénoxy benzyl | 86/14 | 43 % |
| $C_6H_5SO_2$ | Et | $CH_3$ | 33/66 | 70 % |
| $C_6H_5SO_2$ | Me | 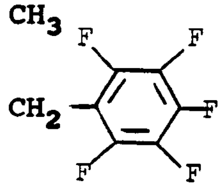 | 34/64 | 73 % |
| $CH_3SO_2$ | tbu | $CH_3$ | 14/86 | 45 % |
| et en série 1R trans | | | | |
| $C_6H_5SO$ | tbu | 3-phénoxy benzyl | 100/0 | 50 % |

Les conditions opératoires sont les suivantes :
1 mmole de produit 1, 2 équivalents de $Na_2S_2O_4$ (titre 75 - 80 %) 2 équivalents de $NaHCO_3$, 2 équivalents de chlorure de tricaprylméthylammonium, 5 cm³ d'eau, 5 cm³ de cyclohexane ; temps de reflux : 1 heure.

PREPARATION 15 : (tert-butylsulfonyl) acétate de tert-butyle.

Stade A : (tert-butylthio) acétate de tert-butyle.

On refroidit à + 10°C une solution renfermant 9 g de tert-butylthiol et 100 ml de tétrahydrofuranne. On ajoute en 15 minutes, 11,2 g de tert-butylate de potassium en solution dans 150 ml de tétrahydrofuranne. On agite pendant 5 minutes à + 10°C et ajoute en 15 minutes à la même température 19,5 g de bromoacétate de tert-butyle en solution dans 50 ml de tétrahydrofuranne. On agite pendant 1 heure à 20°C, ajoute 75 ml d'eau et sature en chlorure de sodium. On décante la phase organique, la sèche et la concentre sous pression réduite. On obtient 20 g de produit recherché que l'on utilise tel quel dans le stade suivant.

Stade B : (tert-butylsulfonyl) acétate de tert-butyle.

On introduit 92 g d'oxone en solution dans 400 ml d'eau dans une solution renfermant 20 g de produit préparé au stade A dans 300 ml de méthanol. On agite 18 heures à 20°C. On évapore le méthanol sous pression réduite. On extrait la phase aqueuse au chlorure de méthylène. On sèche et concentre. On obtient 21,5 g de sulfone fondant à 70°C.

## Revendications

1. Procédé de préparation des produits formule (V) :

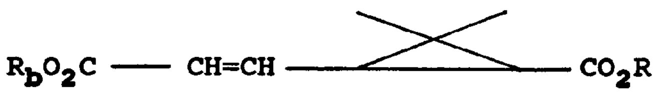

(V)

dans laquelle $R_b$ représente un radical alkyle linéaire, ramifié ou cyclisé saturé ou insaturé, renfermant jusqu'à 18 atomes de carbone, éventuellement substitué par un ou plusieurs groupes fonctionnels, ou $R_b$ représente un radical aryle renfermant jusqu'à 14 atomes de carbone, éventuellement substitué par un ou plusieurs groupes fonctionnels et R représente un atome d'hydrogène, un radical alkyle renfermant jusqu'à 8 atomes de carbone ou

- soit un groupe

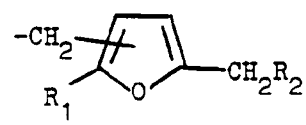

dans lequel le substituant $R_1$ représente un atome d'hydrogène ou un radical méthyle et le substituant $R_2$ un aryle monocyclique ou un groupe 2-propynyle et notamment un groupe (5-benzyl 3-furyl) méthyle,

- soit un groupe

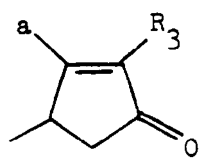

dans lequel a représente un atome d'hydrogène ou un radical méthyle et $R_3$ représente un radical organique aliphatique comportant de 2 à 6 atomes de carbones et une ou plusieurs insaturations carbone-carbone et notamment le radical $-CH_2-CH=CH_2$, $-CH_2-CH=CH-CH_3$, $-CH_2-CH=CH-CH=CH_2$, $-CH_2-CH=CH-CH_2-CH_3$,

- soit un groupe

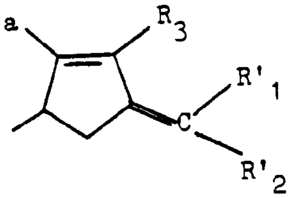

dans lequel a représente un atome d'hydrogène ou un radical méthyle, $R_3$ conserve la même signification que précédemment, $R'_1$ et $R'_2$, identiques ou différents, représentent un atome d'hydrogène, un atome d'halogène, un radical alkyle renfermant de 1 à 6 atomes de carbone, un radical aryle comportant de 6 à 10 atomes de carbone, un groupe alkyloxycarbonyle comportant de 2 à 5 atomes de carbone,

- soit un groupe

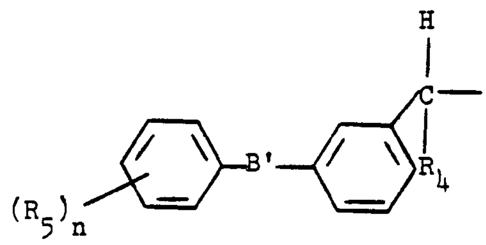

dans lequel B' représente un atome d'oxygène ou de soufre, un groupe carbonyle, méthylène, sulfinyle ou sulfonyle et $R_4$ représente un atome d'hydrogène, un radical cyano, un radical méthyle, un radical carbamoyle, un radical thiocarbamoyle ou un radical éthynyle, $R_5$ représente un atome d'halogène ou un radical méthyle et n représente un nombre égal à 0, 1 ou 2, et notamment le groupe 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thiocarbamoyle 3-phénoxy benzyle,

- soit un groupe

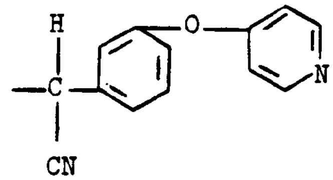

- soit un groupe

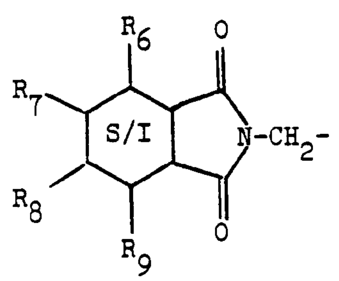

dans lequel les substituants $R_6$, $R_7$, $R_8$, $R_9$ représentent un atome d'hydrogène, un atome de chlore, ou un radical méthyle et dans lequel S/I symbolise un cycle aromatique ou un cycle analogue dihydro ou tétrahydro,

- soit un groupe

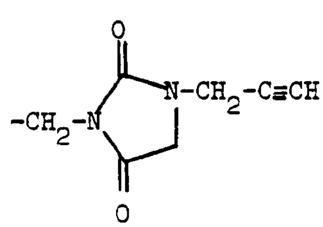

- soit un groupe

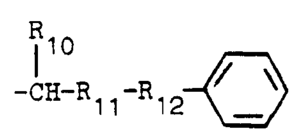

dans lequel $R_{10}$ représente un atome d'hydrogène ou un radical cyano, $R_{12}$ représente un radical méthylène ou un atome d'oxygène, $R_{11}$ représente un radical thiazolediyle ou thiadiazolediyle lié à

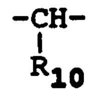

par l'une quelconque des positions disponibles du cycle et à $R_{12}$ par l'atome de carbone compris entre l'atome de soufre et un atome d'azote,

- soit un groupe

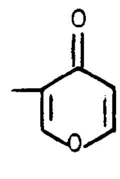

- soit un groupe

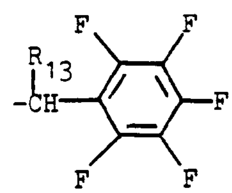

dans lequel $R_{13}$ représente un atome d'hydrogène ou un radical cyano,

- soit un groupe

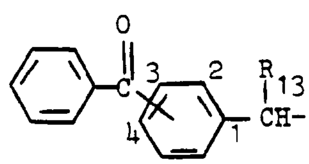

dans lequel $R_{13}$ est défini comme ci-dessus, et le radical benzoyle est en position 3 ou 4,

- soit, un groupe

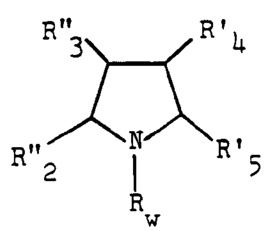

dans lequel l'un des radicaux $R''_2$ ou $R''_3$ représente un radical -CHZ, Z représentant un atome d'hydrogène, un groupe cyano, éthynyle, trifluorométhyle ou un radical alkyle renfermant de 1 à 3 atomes de carbone et celui des radicaux R"2 ou R"3 qui ne représente pas un radical -CHZ ainsi que les radicaux $R'_4$ et $R'_5$ identiques ou différents les uns des autres, représentent un atome d'hydrogène, un atome d'halogène, un radical alkyle renfermant jusqu'à 18 atomes de carbone, un radical aryle renfermant jusqu'à 14 atomes de carbone, un radical aralkyle renfermant jusqu'à 18 atomes de carbone, un radical cyano, un radical trifluorométhyle, un radical alcoxycarbonyle renfermant jusqu'à 8 atomes de carbone, un groupe nitro, un radical alkyloxy renfermant jusqu'à 8 atomes de carbone,

un radical :

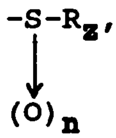 ou 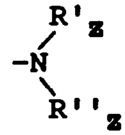 ,

n étant égal à 0,1 ou 2 et les radicaux $R_z$, $R'_z$ et $R''_z$ représentant un radical alkyle renfermant de 1 à 8 atomes de carbone, les radicaux $R'_4$ et $R'_5$ pouvant aussi former un homocycle carboné saturé ou insaturé renfermant jusqu'à 8 atomes de carbone et $R_w$ représente :

- soit un radical

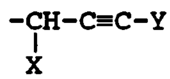

dans lequel X et Y, identiques ou différents l'un de l'autre, représentent un atome d'hydrogène, un atome d'halogène, un radical alkyle renfermant de 1 à 8 atomes de carbone ou un radical aryle renfermant jusqu'à 14 atomes de carbone

- soit un radical

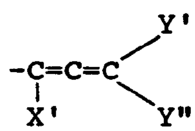

ou
un radical

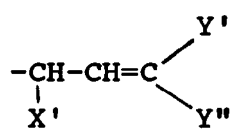

dans lequel X', Y' et Y'', identiques ou différents les uns des autres, représentent l'une des valeurs indiquées ci-dessus pour X et Y ;

- soit un radical

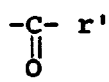

dans lequel r' représente un atome d'hydrogène, un radical alkyle renfermant jusqu'à 14 atomes de carbone, un radical aralkyle renfermant jusqu'à 18 atomes de carbone, un radical trifluorométhyle, un radical alcoxycarbonyle, un radical alkyloxy renfermant jusqu'à 8 atomes de carbone, un radical alkylthio

- soit un radical

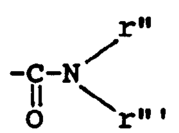 ,

dans lequel r" et r‴, identiques ou différents l'un de l'autre, représentent un atome d'hydrogène, un radical alkyle renfermant de 1 à 18 atomes de carbone, un radical aryle renfermant jusqu'à 14 atomes de carbone, un radical aralkyle renfermant jusqu'à 18 atomes de carbone, un radical trifluorométhyle, un radical alcoxycarbonyle renfermant jusqu'à 8 atomes de carbone ou un radical alkyloxy renfermant jusqu'à 8 atomes de carbone.

- soit un groupe

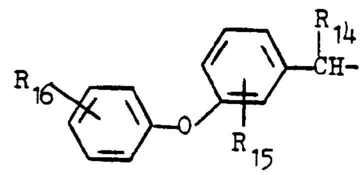

dans lequel $R_{14}$ représente un atome d'hydrogène, un radical méthyle, éthynyle ou cyano et $R_{15}$ et $R_{16}$, différents, représentent un atome d'hydrogène, de fluor ou de brome,

- soit un groupe

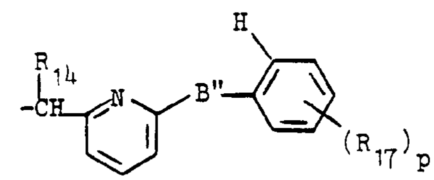

dans lequel $R_{14}$ est défini comme précédemment, B" représente un atome d'oxygène ou de soufre, p est un nombre égal à 0, 1 ou 2, $R_{17}$ représente un groupe alkyle renfermant de 1 à 4 atomes de carbone, alkylthio renfermant de 1 à 4 atomes de carbone, alkylsulfonyle renfermant de 1 à 4 atomes de carbone, trifluorométhyle, chloro, fluoro ou bromo et quand p est égal à 2, soit $(R_{17})_p$ représente un groupe méthylènedioxy lié au noyau phényle aux positions 3 et 4, soit chacun des $R_{17}$ représente indépendamment un des groupes cités ci-dessus, caractérisée en ce que l'on soumet un composé de formule (I) :

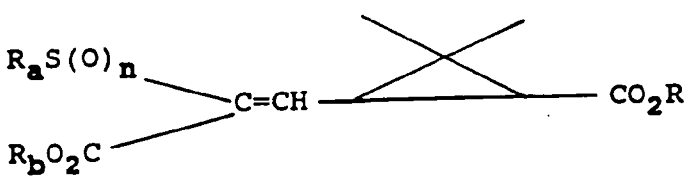

(I)

dans laquelle n représente le nombre 0, 1 ou 2, $R_a$ représente un radical alkyle linéaire, ramifié ou cyclisé, saturé ou insaturé, renfermant jusqu'à 18 atomes de carbone ou un radical aryle renfermant jusqu'à 14 atomes de carbone, éventuellement substitué par un ou plusieurs groupes fonctionnels, $R_b$ et R sont tels que définis ci-dessus, à l'action de l'anion $S_2O_4^{--}$ ou toute autre source de $HSO_2^-$ ou $SO_2^{--}$, pour obtenir le composé de formule (IV) correspondant :

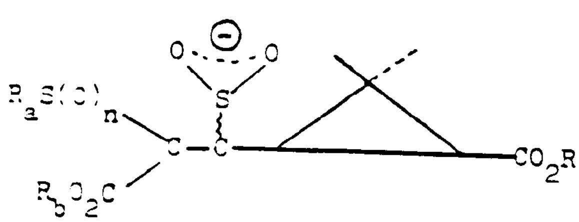

(IV)

dans laquelle $R_a$, $R_b$ et R conservent la même signification que précédemment, que l'on isole si désiré ou qui se décompose spontanément pour donner le composé de formule (V) correspondant.

2. Procédé selon la revendication 1, caractérisé par le fait que dans les composés de formule (I) tels que

définis à la revendication 1, n représente le nombre 2.

3. Procédé selon la revendication 1 caractérisé par le fait que dans les composés de formule (I) tels que définir à la revendication 1 ou 2, $R_a$ représente un radical tert-butyle.

4. Procédé selon la revendication 1, caractérisé par le fait que dans les composés de formule (I) tels que définis à la revendication 1 ou 2, $R_a$ représente un radical phényle, éventuellement substitué par un radical $Oalc_1$, $N(alc_2)_2$, $alc_3$, $alc_1$, $alc_2$ et $alc_3$ représentant un radical alkyle renfermant jusqu'à 8 atomes de carbone.

5. Procédé selon la revendication 1, caractérisé par le fait que dans les composés de formule (I) tels que définis à l'une quelconque des revendications 1 à 4, $R_b$ représente un radical alkyle linéaire ou ramifié, saturé, renfermant jusqu'à 6 atomes de carbone, éventuellement substitué par un ou plusieurs atomes d'halogène.

6. Procédé selon la revendication 1, caractérisé par le fait que dans les composés de formule (I) tels que définis à la revendication 5, $R_b$ représente un radical tert-butyle ou un radical

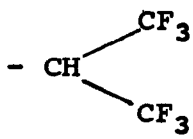

7. Procédé selon la revendication 1, caractérisé par le fait que dans les composés de formule (I) tels que définis à l'une quelconque des revendications 1 à 6, R représente un radical alkyle linéaire ou ramifié, saturé, renfermant jusqu'à 4 atomes de carbone.

8. Procédé selon la revendication 1, caractérisé par le fait que dans les composés de formule (I), tels que définis à l'une quelconque des revendications 1 à 7, R représente un radical

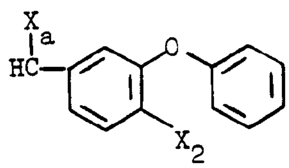

dans lesquels $X_a$ représente un atome d'hydrogène, un radical méthyle, cyano ou éthynyle, et $X_2$ représente un atome d'hydrogène ou un atome de fluor.

9. Procédé selon la revendication 1, caractérisé par le fait que dans les composés de formule (I), tels que définis à l'une quelconque des revendications 1 à 8, R représente un radical

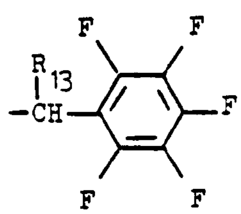

dans lequel $R_{13}$ conserve la même signification que dans la revendication 1.

10. Procédé selon la revendication 1, caractérisé par le fait que dans les composés de formule (I) tels que définis à l'une quelconque des revendications 1 à 9, la structure stérique au niveau du groupe cyclique 2,2-diméthyl cyclopropane est (1R cis).

**11.** Procédé selon la revendication 1 des produits de formule (I) tel que défini à l'une quelconque des revendications 1 à 10, caractérisé en ce que l'agent réducteur est le dithionite de sodium, $Na_2S_2O_4$, dans un mélange d'eau et de solvant organique avec éventuellement un agent de transfert de phase.

**12.** A titre de produit intermédiaire, le produit de formule (IV) telle que définie à la revendication 1.

**Patentansprüche**

**1.** Verfahren zur Herstellung der Produkte der Formel (V)

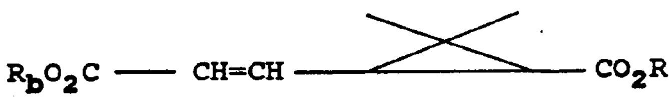

(V)

worin $R_b$ einen gesättigten oder ungesättigten, linearen, verzweigten oder cyclischen Alkylrest mit bis zu 18 Kohlenstoffatomen, der gegebenenfalls durch eine oder mehrere funktionelle Gruppen substituiert ist, bedeutet, oder $R_b$ einen Arylrest mit bis zu 14 Kohlenstoffatomen, der gegebenenfalls durch eine oder mehrere funktionelle Gruppen substituiert ist, bedeutet, und R wiedergibt: ein Wasserstoffatom, einen Alkylrest mit bis zu 8 Kohlenstoffatomen oder

- entweder eine Gruppe

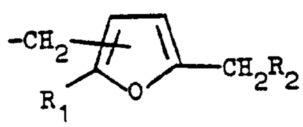

worin der Substituent $R_1$ ein Wasserstoffatom oder einen Methylrest bedeutet, und der Substituent $R_2$ einen monocyclischen Arylrest oder eine 2-Propinylgruppe bedeutet,und insbesondere eine (5-Benzyl-3-furyl)-methylgruppe.

- oder eine Gruppe

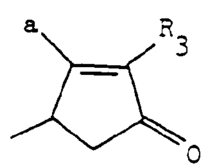

worin a für ein Wasserstoffatom oder eine Methylgruppe steht, und $R_3$ einen aliphatischen, organischen Rest mit 2 bis 6 Kohlenstoffatomen und einer oder mehreren Kohlenstoff-Kohlenstoff-Unsättigungen und insbesondere den Rest $-CH_2-CH=CH_2$, $-CH_2-CH=CH-CH_3$, $-CH_2-CH=CH-CH=CH_2$, $-CH_2-CH=CH-CH_2-CH_3$ bedeutet,

- oder eine Gruppe

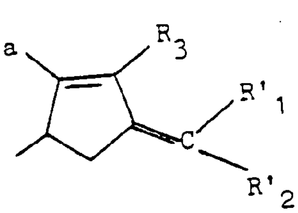

worin a für ein Wasserstoffatom oder eine Methylgruppe steht, $R_3$ die vorstehend angegebene Bedeutung besitzt, $R'_1$ und $R'_2$, identisch oder voneinander verschieden, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen bedeuten,

- oder eine Gruppe

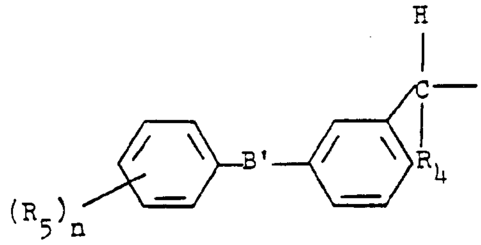

worin B' für ein Sauerstoff- oder Schwefelatom, eine Carbonyl-, Methylen-, Sulfinyl- oder Sulfonylgruppe steht, und $R_4$ ein Wasserstoffatom, eine Cyanogruppe, einen Methylrest, einen Carbamoylrest, einen Thiocarbamoylrest oder einen Ethinylrest bedeutet, $R_5$ ein Halogenatom oder einen Methylrest wiedergibt, und n für eine Zahl entsprechend 0, 1 oder 2 steht, und insbesondere die 3-Phenoxybenzyl-, α-Cyano-3-phenoxybenzyl-, α-Ethinyl-3-phenoxybenzyl-, 3-Benzoylbenzyl-, 1-(3-Phenoxyphenyl)-ethyl oder α-Thiocarbamoyl-3-phenoxybenzylgruppe,

- oder eine Gruppe

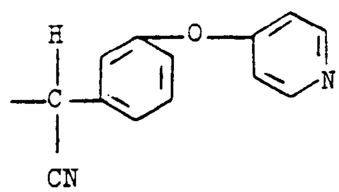

- oder eine Gruppe

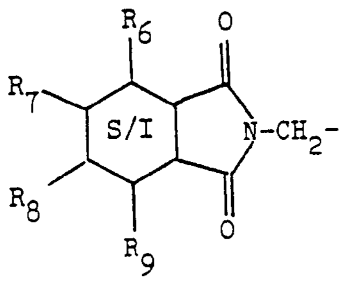

worin die Substituenten $R_5$, $R_7$, $R_8$, $R_9$ ein Wasserstoffatom, ein Chloratom oder eine Methylgruppe darstellen, und worin S/I einen aromatischen Ring oder einen analogen Dihydro- oder Tetrahydroring symbolisiert,

- oder eine Gruppe

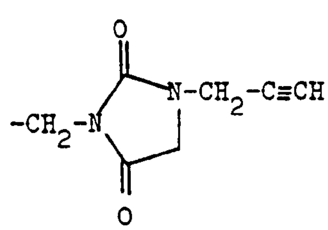

- oder eine Gruppe

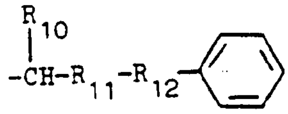

worin $R_{10}$ für ein Wasserstoffatom oder eine Cyanogruppe steht, $R_{12}$ einen Methylenrest oder ein Sauerstoffatom bedeutet, $R_{11}$ einen Thiazoldiyl- oder Thiadiazoldiylrest, der an

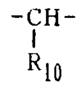

über irgendeine der verfügbaren Positionen des Rings und an $R_{12}$ durch das Kohlenstoffatom zwischen dem Schwefelatom und einem Stickstoffatom gebunden ist, bedeutet,

- oder eine Gruppe

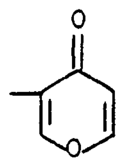

- oder eine Gruppe

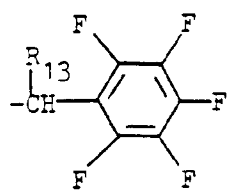

worin $R_{13}$ für ein Wasserstoffatom oder eine Cyanogruppe steht,

- oder eine Gruppe

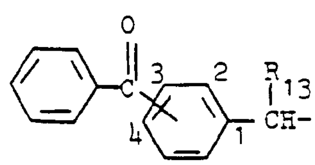

worin $R_{13}$ wir vorstehend definiert ist, und der Benzoylrest sich in 3- oder 4-Stellung befindet,

- oder eine Gruppe

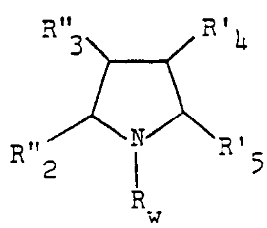

worin einer der Reste $R''_2$ oder $R''_3$ einen Rest -CHZ wiedergibt, wobei Z ein Wasserstoffatom, eine Cyano-, Ethinyl-, Trifluoromethylgruppe oder einen Alkylrest mit 1 bis 3 Kohlenstoffatomen bedeutet,

und derjenige der Reste $R''_2$ oder $R''_3$, der nicht einen Rest -CHZ wiedergibt, sowie die Reste $R'_4$ und $R'_5$, identisch oder voneinander verschieden, ein Wasserstoffatom, ein Halogenatom, einen Alkylrest mit bis zu 18 Kohlenstoffatomen, einen Arylrest mit bis zu 14 Kohlenstoffatomen, einen Aralkylrest mit bis zu 18 Kohlenstoffatomen, eine Cyanogruppe, eine Trifluormethylgruppe, einen Alkoxycarbonylrest mit bis zu 8 Kohlenstoffatomen, eine Nitrogruppe, einen Alkyloxyrest mit bis zu 8 Kohlenstoffatomen, einen Rest

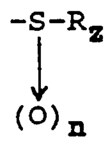 oder 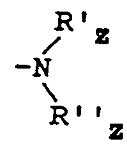 ,

worin n für 0, 1 oder 2 steht, und die Reste $R_Z$, $R'_Z$ und $R''_Z$ einen Alkylrest mit 1 bis 8 Kohlenstoffatomen bedeuten, wobei die Reste $R'_4$ und $R'_5$ auch einen gesättigten oder ungesättigten Kohlenstoff-Homocyclus mit bis zu 8 Kohlenstoffatomen bilden können, bedeuten, und $R_W$ bedeutet:

- entweder einen Rest

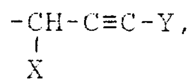

worin X und Y, identisch oder voneinander verschieden, für ein Wasserstoffatom, ein Halogenatom, einen Alkylrest mit 1 bis 8 Kohlenstoffatomen oder einen Arylrest mit bis zu 14 Kohlenstoffatomen stehen,

- oder einen Rest

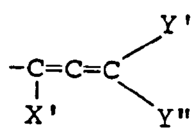

oder
einen Rest

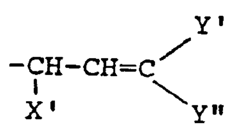

worin X', Y' und Y'', identisch oder voneinander verschieden, eine der vorstehend für X und Y angegebenen Bedeutungen wiedergeben,

- oder einen Rest

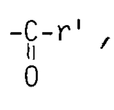

worin r' für ein Wasserstoffatom, einen Alkylrest mit bis zu 14 Kohlenstoffatomen, einen Aralkylrest mit bis zu 18 Kohlenstoffatomen, eine Trifluormethylgruppe, einen Alkoxycarbonylrest, einen Alkyloxyrest mit bis zu 8 Kohlenstoffatomen, einen Alkylthiorest steht,

- oder einen Rest

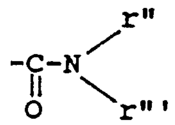

,

worin r" und r"', identisch oder voneinander verschieden, für ein Wasserstoffatom, einen Alkylrest mit 1 bis 18 Kohlenstoffatomen, einen Arylrest mit bis zu 14 Kohlenstoffatomen, einen Aralkylrest mit bis zu 18 Kohlenstoffatomen, eine Trifluormethylgruppe, einen Alkoxycarbonylrest mit bis zu 8 Kohlenstoffatomen oder einen Alkyloxyrest mit bis zu 8 Kohlenstoffatomen stehen,

- oder eine Gruppe

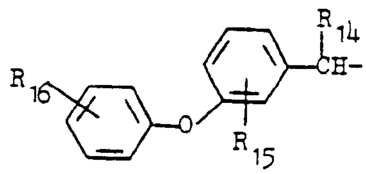

worin $R_{14}$ ein Wasserstoffatom, eine Methyl-, Ethinyl- oder Cyanogruppe bedeutet, und $R_{15}$ und $R_{16}$, voneinander verschieden, für ein Wasserstoff-, Fluor- oder Bromatom stehen,

- oder eine Gruppe

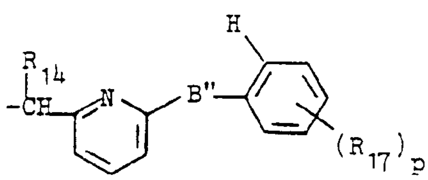

worin $R_{14}$ wie vorstehend definiert ist, B" für ein Sauerstoff- oder Schwefelatom steht, p eine Zahl entsprechend 0, 1 oder 2 ist, $R_{17}$ eine Alkylgruppe mit 1 bis 4 Kohlenstoffatomen, Alkylthio mit 1 bis 4 Kohlenstoffatomen, Alkylsulfonyl mit 1 bis 4 Kohlenstoffatomen, Trifluormethyl, Chlor, Fluor oder Brom bedeutet, und wenn p für 2 steht, entweder $(R_{17})_p$ eine Methylendioxygruppe, die an den Phenylring über die Positionen 3 und 4 gebunden ist, bedeutet, oder ein jedes von $R_{17}$ unabhängig eine der vorstehend angegebenen Gruppen darstellt, dadurch gekennzeichnet, daß man eine Verbindung der Formel (I)

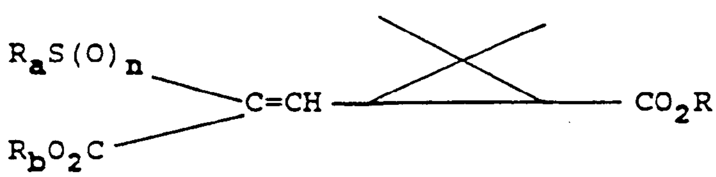

(I)

worin n die Zahl 0, 1 oder 2 bedeutet, $R_a$ für einen gesättigten oder ungesättigten, linearen, verzweigten oder cyclischen Alkylrest mit bis zu 18 Kohlenstoffatomen oder einem Arylrest mit bis zu 14 Kohlenstoffatomen, der gegebenenfalls durch eine oder mehrere funktionelle Gruppen substituiert ist, steht, $R_b$ und R wie vorstehend definiert sind, der Einwirkung des Anions $S_2O_4^{--}$ oder jeder anderen Quelle für $HSO_2^-$ oder $SO_2^{--}$ unterzieht, um zu der entsprechenden Verbindung der Formel (IV)

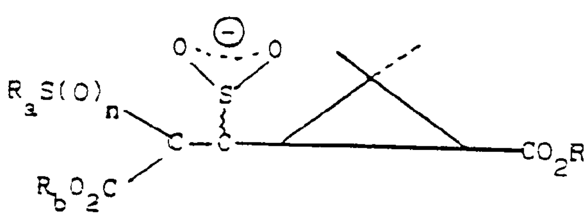

(IV)

zu gelangen, worin $R_a$, $R_b$ und R die vorstehend angegebene Bedeutung besitzen, welche man gewünschtenfalls isoliert oder die sich spontan zersetzt, um die entsprechende Verbindung der Formel (V) zu ergeben.

2. Verfahren gemäß Anspruch 1, dadurch gekennzeichnet, daß in den Verbindungen der Formel (I), wie in Anspruch 1 definiert, n die Zahl 2 bedeutet.

3. Verfahren gemäß Anspruch 1, dadurch gekennzeichnet, daß in den Verbindungen der Formel (I), wie in Anspruch 1 oder 2 definiert, $R_a$ einen tert.-Butylrest bedeutet.

4. Verfahren gemäß Anspruch 1, dadurch gekennzeichnet, daß in den Verbindungen der Formel (I), wie in Anspruch 1 oder 2 definiert, $R_a$ einen Phenylrest bedeutet, der gegebenenfalls durch einen Rest $Oalc_1$, $N(alc_2)_2$, $alc_3$ substituiert ist, wobei $alc_1$, $alc_2$ und $alc_3$ einen Alkylrest mit bis zu 8 Kohlenstoffatomen wiedergeben.

5. Verfahren gemäß Anspruch 1, dadurch gekennzeichnet, daß in den Verbindungen der Formel (I), wie in einem der Ansprüche 1 bis 4 definiert, $R_b$ für einen gesättigten, linearen oder verzweigten Alkylrest mit bis zu 6 Kohlenstoffatomen steht, der gegebenenfalls durch ein oder mehrere Halogenatome substituiert ist.

6. Verfahren gemäß Anspruch 1, dadurch gekennzeichnet, daß in den Verbindungen der Formel (I), wie in Anspruch 5 definiert, $R_b$ für einen tert.-Butylrest oder einen Rest

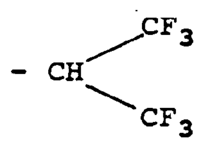

steht.

7. Verfahren gemäß Anspruch 1, dadurch gekennzeichnet, daß in den Verbindungen der Formel (I), wie in einem der Ansprüche 1 bis 6 definiert, R für einen gesättigten, linearen oder verzweigten Alkylrest mit bis zu 4 Kohlenstoffatomen steht.

8. Verfahren gemäß Anspruch 1, dadurch gekennzeichnet, daß in den Verbindungen der Formel (I), wie in einem der Ansprüche 1 bis 7 definiert, R für einen Rest

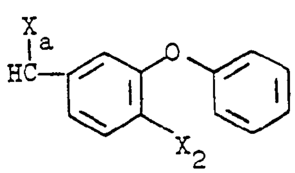

steht, worin $X_a$ ein Wasserstoffatom, eine Methyl-, Cyano- oder Ethinylgruppe bedeutet, und $X_2$ ein Wasserstoffatom oder ein Fluoratom wiedergibt.

9. Verfahren gemäß Anspruch 1, dadurch gekennzeichnet, daß in den Verbindungen der Formel (I), wie in einem der Ansprüche 1 bis 8 definiert, R einen Rest bedeutet,

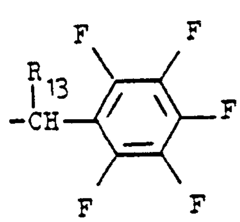

worin $R_{13}$ die in Anspruch 1 angegebene Bedeutung besitzt.

**10.** Verfahren gemäß Anspruch 1, dadurch gekennzeichnet, daß in den Verbindungen der Formel (I), wie in einem der Ansprüche 1 bis 9 definiert, die sterische Struktur im Bereich der cyclischen 2-2-Dimethylcyclopropangruppe die (1R,cis)-Struktur ist.

**11.** Verfahren gemäß Anspruch 1 zur Herstellung von Produkten der Formel (I), wie in einem der Ansprüche 1 bis 10 definiert, dadurch gekennzeichnet, daß das Reduktionsmittel Natrium-Dithionit, $N_2S_2O_4$, in einem Gemisch von Wasser und organischem Lösungsmittel gegebenenfalls mit einem Phasentransfermittel ist.

**12.** Als Zwischenprodukt das Produkt der Formel (IV), wie in Anspruch 1 definiert.

## Claims

**1.** Preparation process for the products of formula (V):

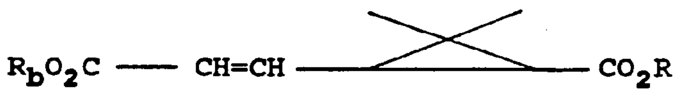

(V)

in which $R_b$ represents a saturated or unsaturated, linear, branched or cyclic alkyl radical containing up to 18 carbon atoms, optionally substituted by one or more functional groups, or $R_b$ represents an aryl radical containing up to 14 carbon atoms, optionally substituted by one or more functional groups, and R represents a hydrogen atom, an alkyl radical containing up to 8 carbon atoms or

- either a group

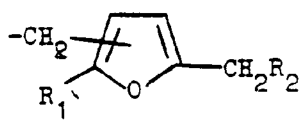

in which the $R_1$ substituent represents a hydrogen atom or a methyl radical and the $R_2$ substituent represents a monocyclic aryl or a 2-propynyl group and in particular a (5-benzyl 3-furyl) methyl group,

- or a group

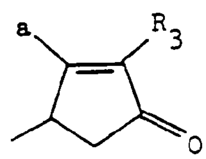

in which a represents a hydrogen atom or a methyl radical and $R_3$ represents an aliphatic organic radical containing 2 to 6 carbon atoms and one or more carbon-carbon unsaturations and in particular one of the following radicals: $-CH_2-CH=CH_2$, $-CH_2-CH=CH-CH_3$, $-CH_2-CH=CH-CH=CH_2$, $-CH_2-CH=CH-CH_2-CH_3$,

- or a group

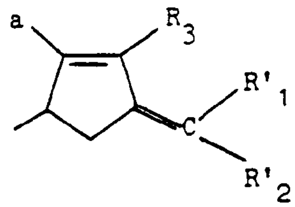

in which a represents a hydrogen atom or a methyl radical, $R_3$ keeps the same meaning as previously, $R'_1$ and $R'_2$, identical or different, represent a hydrogen atom, a halogen atom, an alkyl radical containing 1 t 6 carbon atoms, an aryl radical containing 6 to 10 carbon atoms, an alkyloxycarbonyl group containing 2 to 5 carbon atoms,

- or a group

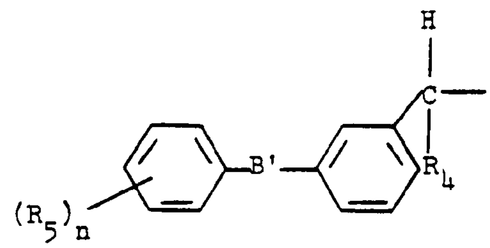

in which B' represents an oxygen or sulphur atom, a carbonyl, methylene, sulphinyl or sulphonyl group and $R_4$ represents a hydrogen atom, a cyano radical, a methyl radical, a carbamoyl radical, a thiocarbamoyl radical or an ethynyl radical, $R_5$ represents a halogen atom or a methyl radical and n represents a number equal to 0, 1 or 2, and in particular one of the following groups: 3-phenoxy benzyl, alpha-cyano 3-phenoxy benzyl, alpha-ethynyl 3-phenoxy benzyl, 3-benzoyl benzyl, 1-(3-phenoxy phenyl) ethyl or alpha-thiocarbamoyl 3-phenoxy benzyl,

- or a group

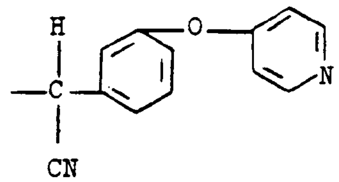

- or a group

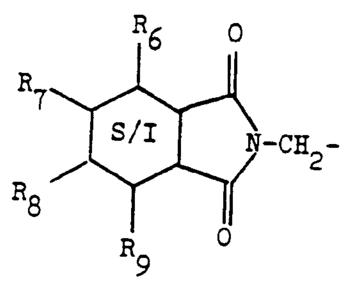

in which the $R_6$, $R_7$, $R_5$, $R_9$ substituents represent a hydrogen atom, a chlorine atom, or a methyl radical and in which S/I symbolizes an aromatic ring or a similar dihydro or tetrahydro ring,

- or a group

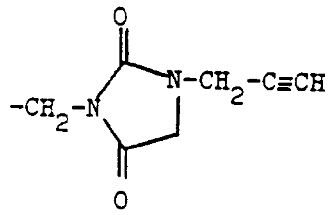

- or a group

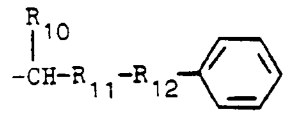

in which $R_{10}$ represents a hydrogen atom or a cyano radical, $R_{12}$ represents a methylene radical or an oxygen atom, $R_{11}$ represents a thiazolediyl or thiadiazolediyl radical linked to

$$-\underset{\displaystyle R_{10}}{\overset{|}{C}H}-$$

by any of the available positions of the ring and to $R_{12}$ by the carbon atom between the sulphur atom and a nitrogen atom,

- or a group

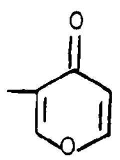

- or a group

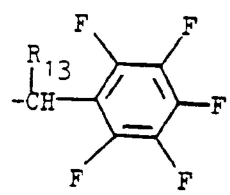

in which $R_{13}$ represents a hydrogen atom or a cyano radical,

- or a group

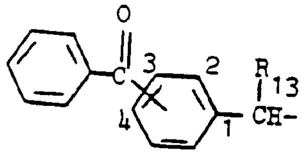

in which $R_{13}$ is defined as above, and the benzoyl radical is in position 3 or 4,

- or a group

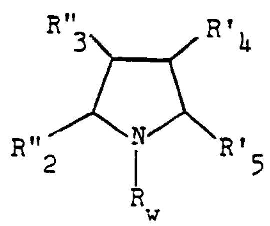

in which one of the $R''_2$ or $R''_3$ radicals represents a -CHZ radical, Z representing a hydrogen atom, a cyano, ethynyl or trifluoromethyl group or an alkyl radical containing 1 to 3 carbon atoms and whichever of the $R''_2$ or $R''_3$ radicals does not represent a -CHZ radical as well as the $R'_4$ and $R'_5$ radical, identical or different, represent a hydrogen atom, a halogen atom, an alkyl radical containing up to 18 carbon atoms, an aryl radical containing up to 14 carbon atoms, an aralkyl radical containing up to 18 carbon atoms, a cyano radical, a trifluoromethyl radical, an alkoxycarbonyl radical containing up to 8 carbon atoms, a nitro group, an alkyloxy radical containing up to 8 carbon atoms, a radical:

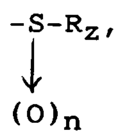 , or 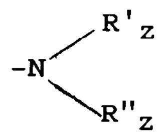 ,

n being equal to 0, 1 or 2 and the $R_z$, $R'_z$ and $R''_z$ radicals representing an alkyl radical containing 1 to 8 carbon atoms, the $R'_4$ and $R'_5$ radical also being able to form a saturated or unsaturated carbonated homocycle containing up to 8 carbon atoms and $R_w$ represents:

- either a

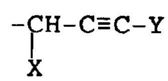

radical in which X and Y, identical or different, represent a hydrogen atom, a halogen atom, an alkyl radical containing 1 to 8 carbon atoms or an aryl radical containing up to 14 carbon atoms

- or a

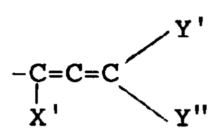

radical or
a

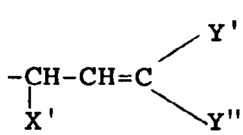

radical
in which X', Y' and Y'', identical or different, represent one of the values indicated above for X and Y;

- or a

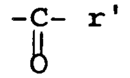

radical in which r' represents a hydrogen atom, an alkyl radical containing up to 14 carbon atoms, an aralkyl radical containing up to 18 carbon atoms, a trifluoromethyl radical, an alkoxycarbonyl radical, an alkyloxy radical containing up to 8 carbon atoms, an alkylthio radical

- or a

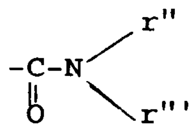

radical, in which r'' and r''', identical or different, represent a hydrogen atom, an alkyl radical containing 1 to 18 carbon atoms, an aryl radical containing up to 14 carbon atoms, an aralkyl radical containing up to 18 carbon atoms, a trifluoromethyl radical, an alkoxycarbonyl radical containing up to 8 carbon atoms or an alkyloxy radical containing up to 8 carbon atoms,

- or a group

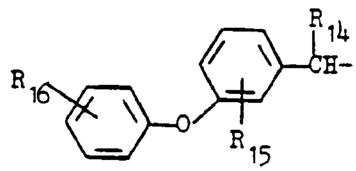

in which $R_{14}$ represents a hydrogen atom, a methyl, ethynyl or cyano radical and $R_{15}$ and $R_{16}$, being different, represent a hydrogen, fluorine or bromine atom,

- or a group

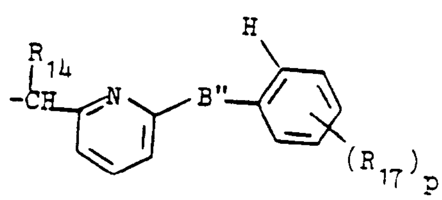

in which $R_{14}$ is defined as previously, B'' represents an oxygen or sulphur atom, p is a number equal to 0, 1 or 2, $R_{17}$ represents an alkyl group containing 1 to 4 carbon atoms, an alkylthio group containing 1 to 4 carbon atoms, an alkylsulphonyl group containing 1 to 4 carbon atoms, a trifluoromethyl, chloro, fluoro or bromo group and when p is equal to 2, either $(R_{17})_p$ represents a methylenedioxy group linked to the phenyl nucleus in positions 3 and 4, or each of the $R_{17}$'s represents independently one of the above-mentioned groups, characterized in that a compound of formula (I):

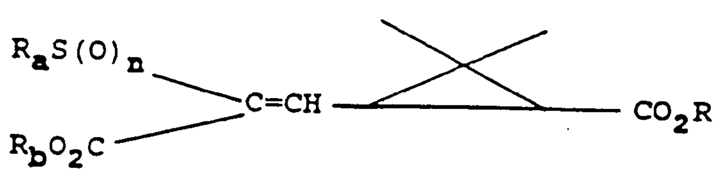

(I)

in which n represents the number 0, 1 or 2, $R_a$ represents a saturated or unsaturated, linear, branched or cyclic alkyl radical containing up to 18 carbon atoms or an aryl radical containing up to 14 carbon atoms, optionally substituted by one or more functional groups, $R_b$ and R are as defined above, is subjected to the action of an $S_2O_4^{--}$ anion or any other source of $HSO_2^{-}$ or $SO_2^{--}$, in order to obtain

the corresponding compound of formula (IV):

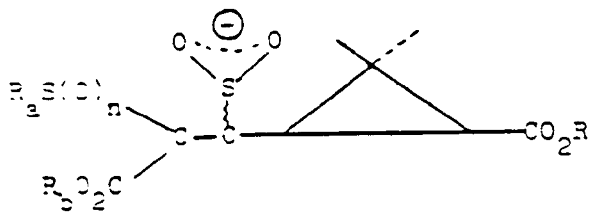

(IV)

in which $R_a$, $R_b$ and R keep the same meaning as previously, which if desired is isolated or which decomposes spontaneously to give the corresponding compound of formula (v).

2. Process according to claim 1, characterized in that in the compounds of formula (I) as defined in claim 1, n represents the number 2.

3. Process according to claim 1, characterized in that in the compounds of formula (I) as defined in claim 1 or 2, $R_a$ represents a tert-butyl radical.

4. Process according to claim 1, characterized in that in the compounds of formula (I) as defined in claim 1 or 2, $R_a$ represents a phenyl radical, optionally substituted by an $Oalk_1$, $N(alk_2)_2$, $alk_3$ radical, $alk_1$, $alk_2$ and $alk_3$ representing an alkyl radical containing up to 8 carbon atoms.

5. Process according to claim 1, characterized in that in the compounds of formula (I) as defined in any one of claims 1 to 4, $R_b$ represents a saturated, linear or branched alkyl radical containing up to 6 carbon atoms, optionally substituted by one or more halogen atoms.

6. Process according to claim 1, characterized in that in the compounds of formula (I) as defined in claim 5, $R_b$ represents a tert-butyl radical or a

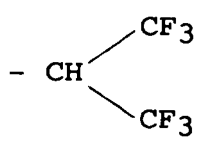

radical.

7. Process according to claim 1, characterized in that in the compounds of formula (I) as defined in any one of claims 1 to 6, R represents a saturated, linear or branched alkyl radical containing up to 4 carbon atoms.

8. Process according to claim 1, characterized in that in the compounds of formula (I), as defined in any one of claims 1 to 7, R represents a radical:

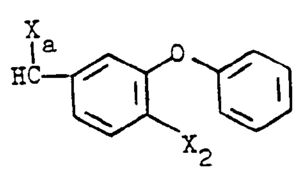

in which $X_a$ represents a hydrogen atom, a methyl, cyano or ethynyl radical, and $X_2$ represents a hydrogen atom or a fluorine atom.

9. Process according to claim 1, characterized in that in the compounds of formula (I), as defined in any one of claims 1 to 8, R represents a radical:

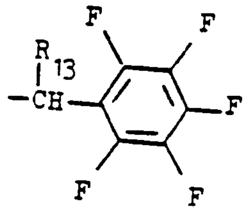

in which $R_{13}$ keeps the same meaning as in claim 1.

**10.** Process according to claim 1, characterized in that in the compounds of formula (I) as defined in any one of claims 1 to 9, the steric structure at the level of the 2,2-dimethyl cyclopropane cyclic group is (1R cis).

**11.** Process according to claim 1 for the products of formula (I) as defined in any one of claims 1 to 10, characterized in that the reducing agent is sodium dithionite, $Na_2S_2O_4$, in a mixture of water and an organic solvent optionally with a phase transfer agent.

**12.** As an intermediate product, the product of formula (IV) as defined in claim 1.